(19) Europäisches Patentamt / European Patent Office / Office européen des brevets

(11) **EP 3 684 322 B1**

(12) **EUROPEAN PATENT SPECIFICATION**

(45) Date of publication and mention of the grant of the patent:
**20.04.2022 Bulletin 2022/16**

(21) Application number: **18773748.1**

(22) Date of filing: **20.09.2018**

(51) International Patent Classification (IPC):
**A61J 3/10** (2006.01)    **G01N 27/22** (2006.01)
**G01N 33/15** (2006.01)

(52) Cooperative Patent Classification (CPC):
**A61J 3/10; G01N 27/221; G01N 33/15**

(86) International application number:
**PCT/EP2018/075415**

(87) International publication number:
**WO 2019/057802 (28.03.2019 Gazette 2019/13)**

(54) **PHARMACEUTICAL MANUFACTURING INSTALLATION AND METHOD OF MANUFACTURING OF A PHARMACEUTICAL PRODUCT**

PHARMAZEUTISCHE PRODUKTIONSEINRICHTUNG UND VERFAHREN ZUR HERSTELLUNG EINES PHARMAZEUTISCHEN PRODUKTS

INSTALLATION DE FABRICATION PHARMACEUTIQUE ET PROCÉDÉ DE FABRICATION D'UN PRODUIT PHARMACEUTIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **21.09.2017 EP 17192423**

(43) Date of publication of application:
**29.07.2020 Bulletin 2020/31**

(73) Proprietor: **F. Hoffmann-La Roche AG**
**4070 Basel (CH)**

(72) Inventors:
• **SIBIK, Juraj**
**4070 Basel (CH)**
• **CHALUS, Pascal**
**4070 Basel (CH)**

(74) Representative: **Latscha Schöllhorn Partner AG**
**Grellingerstrasse 60**
**4052 Basel (CH)**

(56) References cited:
| | |
|---|---|
| DE-A1- 2 502 098 | US-A- 5 135 113 |
| US-A1- 2003 059 614 | US-A1- 2009 026 373 |
| US-A1- 2009 237 090 | |

**Description**

Technical Field

The present invention relates to a pharmaceutical manufacturing installation comprising a processing equipment arranged to provide a pharmaceutical substance in a solid state and a solid fraction sensor, and, furthermore, the invention relates to a method of manufacturing of a pharmaceutical product. Such a device is disclosed in the DE2502098. Z

Background Art

Porosity of solids has a tremendous effect on their mechanical properties and is hence of importance in many industries, including pharmaceutical, chemical or food industry. In pharmaceutical manufacturing, porosity of the intermediates influences also the porosity of the final solid dosage forms, while the porosity of the final dosage forms influence their disintegration and dissolution behaviour. Hence, porosity of the intermediates and final dosage forms plays an important role in the bioavailability of pharmaceutical products.

The intermediate porosity is of particular importance in dry granulation of powder mixtures via roller compaction and in tablet pressing. In roller compaction, the powder mixture is first pressed into a ribbon using two spinning rolls and the ribbon is then milled into granules. Using too small compaction force during the roller compaction can result in fragile granules and high content of small granules, with only limited improvement in the flowability and prevention of segregation in comparison to the input powder mixture. On the other hand, too large compaction force would take away significant part of compressibility of the powder and prohibit further pressing into tablet. Knowledge of ribbon porosity can serve as a good indication for both granule size and tablet mechanical properties of a target pharmaceutical sample. In tablets, too high porosity will likely result in chipping and breaking of the tablet, while too low porosity may negatively affect the release of the drug substance from the tablet.

Commonly, in pharmaceutical manufacturing installations, the porosity of solid state intermediates and final products is determined by off-line analysis. When the true density is known, the bulk porosity can be determined by simple measurements of weight and bulk volume. For more accurate determination of volume for a sample with uneven thickness one often uses surface scanning laser confocal displacement meter. On the other hand, technologies like pycnometry can provide absolute measure of porosity and pore distribution without any prior knowledge, although at a higher labour cost.

Further, there is an on-going search for pharmaceutical manufacturing installations with suitable process analytical technologies (PAT) around manufacturing pharmaceutical products and their intermediates. In particular, it is aimed to achieve processing without any interruption such that the above off-line analysis typically is not appropriate. In this context, some earlier proposals considered utilization of NIR spectroscopy, a common PAT tool which is sensitive to both chemical and physical properties of the sample. However, NIR provides an indirect measure of porosity based on a somewhat impractical multivariate calibration and it is not trivial to isolate the undesired chemical and other physical effects in the porosity prediction. Terahertz spectroscopy provides a more accurate and easier to calibrate alternative, but is still relatively new to pharmaceutical industry and requires further design for implementation as PAT tool. Recently, a novel low-cost measurement based on thermal imaging has been proposed as a solution for ribbon porosity analysis during roller compaction. It is however suitable only for the ribbons of sufficient quality and it requires careful consideration of environmental effects. All of the aforementioned techniques are however still comparably difficult to adapt for inline /online automated measurements in pharmaceutical manufacturing installations that could be used as process analytical technology.

Therefore, an object of the invention is to further develop a pharmaceutical manufacturing installation or method to provide for suitable inline /online automated measurements.

Disclosure of the Invention

According to the invention this need is settled by a pharmaceutical manufacturing installation with the features of independent claim 1 and by method of manufacturing a pharmaceutical product with the features of independent claim 8. Preferred embodiments are subject of the dependent claims.

In particular, in one aspect of the invention, a pharmaceutical manufacturing installation comprises a processing equipment arranged to provide a pharmaceutical substance in a solid state and a solid fraction sensor. The solid fraction sensor has a first conductor element, a second conductor element, an operation space, an energy source arranged to generate an electric field in the operation space by means of the first conductor element and the second conductor element, and a controller adapted to determine a capacitance between the first and second conductor elements with the pharmaceutical substance in the solid state located in the operation space. The solid fraction sensor is arranged to receive the pharmaceutical substance in its solid state into the operation space by the processing equipment.

The term "drug" as used herein below can relate to a therapeutically active agent, also commonly called active pharmaceutical ingredient (API), as well as to a combination of plural such therapeutically active substances. The term also encompasses diagnostic or imaging agents, like for example contrast agents (e.g. MRI contrast agents), tracers (e.g. PET tracers) and hormones, that need to be administered in liquid form to a patient.

The term "pharmaceutical substance" as used herein can relate to a drug as defined above formulated or reconstituted in a form that is suitable for administration to the patient. For example, besides the drug, a drug or pharmaceutical substance may additionally comprise an excipient and/or other auxiliary ingredients. The pharmaceutical substance can also be a formulation only including one or more excipients and/or other auxiliary ingredients.

The term "drug product" as used herein can relate to a finished end product, comprising a pharmaceutical substance or a plurality of pharmaceutical substances. In particular, a drug product may be a ready to use product having the pharmaceutical substance in an appropriate dosage and/or in an appropriate form for administration. For example, a drug product may include an administration device such as a prefilled syringe or the like. The reference can particularly be similar to the target pharmaceutical sample, wherein, besides the essentially same dielectric properties, the samples can have the same API and/or the essentially same chemistry and/or the essentially same composition.

With the use of solid fraction sensor in the pharmaceutical manufacturing installation according to the invention, it is possible to measure the change in the capacitance of the solid fraction sensor induced by the presence of the pharmaceutical substance. The knowledge of the solid fraction sensor geometry, the pharmaceutical substance geometry and the capacitance change can be used to extract the real part of a dielectric permittivity of the pharmaceutical substance. The measured dielectric permittivity can be calibrated with respect to the pharmaceutical substance's solid fraction allowing a current dielectric sensor to be used as a solid fraction sensor inside a pharmaceutical manufacturing installation. Like this, the solid fraction of the manufactures pharmaceutical substance can efficiently and accurately be determined online or inline.

This setup offers significant practical advantages in comparison to state of the art methods, namely: it is applicable both off-line/at-line as well as inline/online measurement; no electrical contact with the target pharmaceutical sample is needed; and a sensitivity of around less than 3% absolute solid fraction deviation can be achieved. In addition, the possible read-out time can be less than 10 ms, which is fast enough for the desired inline /online application. The use of the current solid fraction sensor can show a good linearity in the target range of interest between 50 and 100% solid fraction for pharmaceutical intermediates and products, such as ribbons and tablets. Furthermore, the measurement can be robust, because it shows low impact of e.g. a product lamination or a product fractionation of the target pharmaceutical sample. Still further, in the setup according to the invention it can be prevented that an electrical contact is required between the pharmaceutical substance and the first or second conductor element.

For an appropriate functioning, the solid fraction sensor preferably is electromagnetically shielded. Like this, disturbances induced by other parts of the manufacturing installation or still other things can be prevented or minimized.

In a preferred embodiment, the processing equipment of the pharmaceutical manufacturing installation comprises a punch arrangement adapted to generate a tablet of the pharmaceutical substance. Thereby, one of the first conductor element and the second conductor element preferably is a part of the punch arrangement of the processing equipment or of a chute or outlet of the processing equipment. Such implementation of one conductor element by one part of the punch arrangement thereof allows for an efficient integration of the solid fraction sensor into the processing equipment. Like this, a compact and efficient arrangement can be achieved.

In another preferred embodiment, the processing equipment comprises two rotatable rolls and a compacting space in between the rolls, wherein the compacting space has a powder inlet zone and a ribbon outlet zone, and the solid fraction sensor is arranged adjacent to the ribbon outlet zone of the compacting space such that a ribbon generated by the two rolls exiting the compacting space is forwarded into the operation space of the solid fraction sensor. Such arrangement allows for efficiently providing information about the solid fraction of the ribbon.

Thereby, the second conductor element preferably is formed as a ribbon support adapted to guide a ribbon exiting the compacting space. Alternatively, one of the two rolls preferably is the second conductor element, wherein the first conductor element can be bent to correspond to the outer surface of the one of the two rolls. Such embodiment of the second conductor element allows for efficiently integrating the solid fraction sensor into the roller compactor pharmaceutical manufacturing installation.

In a preferred embodiment, the controller of the solid fraction sensor comprises calibration data of a reference pharmaceutical substance having the essentially same dielectric properties as the pharmaceutical substance in its solid state, the calibration data of the controller of the solid fraction sensor comprises composition data about the composition of the reference pharmaceutical substance and thickness data about the thickness of the reference pharmaceutical substance, the controller of the solid fraction sensor is adapted to convert the calibration data and the determined capacitance into solid fraction data of the pharmaceutical substance in its solid state, and the controller is adapted to generate a solid fraction signal representing the solid fraction data. In such an embodiment, the solid fraction can automatically be evaluated in a particularly efficient and accurate manner.

The solid fraction signal can be in any suitable form such that information about the solid fraction, i.e. the solid fraction data, is represented. For example, the signal can be an electrical signal, a ultrasonic or other acoustic signal, a (laser) light signal or the like.

Preferably, the controller of the solid fraction sensor has a data storage in which the calibration data is stored. The data storage can be any suitable permanent or volatile data storage such as, e.g., a flash memory, a hard disk, a memory chip, an external storage or cloud storage, or the like.

The calibration data preferably comprises permittivity of the reference pharmaceutical substance and a solid fraction ratio of the reference pharmaceutical substance. Generally, permittivity ($\varepsilon$) or dielectric permittivity can be a measure of resistance that is encountered when forming an electric field in a medium. Relative permittivity can be the factor by which an electric field between charges is decreased relative to vacuum. More specifically, $\varepsilon$ can describe the amount of charge needed to generate one unit of electric flux in the medium. Accordingly, a charge will yield more electric flux in a medium with low $\varepsilon$ than in a medium with high $\varepsilon$. Thus, $\varepsilon$ is the measure of a material's ability to resist an electric field rather than its ability to permit it. Typically, $\varepsilon$ is specified in Farad per meter (F/m). Such information allows an efficient and accurate evaluation of the solid fraction of the target pharmaceutical sample.

Particularly, the calibration data preferably comprises pairs of permittivity and corresponding solid fraction ratio. With such pairs, the permittivity and solid fraction ration can efficiently be interrelated. In particular, the information about the composition of the reference pharmaceutical substance and about the thickness of the reference pharmaceutical substance preferably is a calibration curve. Such a calibration curve allows for an efficient and reproducible evaluation.

Preferably, the energy source of the solid fraction sensor is connected to at least one of the first conductor element and the second conductor element. This allows for an efficient implementation of the sensor. In the same context, the controller preferably is adapted to adjust a strength of the electric field in the operation space.

The first and second conductors of the solid fraction sensor can be made of any suitable conductive material. They can further have any predefined shape or geometry. However, in a preferred and comparably simple embodiment, the first conductor element of the solid fraction sensor and the second conductor element of the solid fraction sensor are metallic and plate-like shaped. The term "plate-like" as used herein can relate to a plate being straight, even or bent. It can also relate to a plane, structured or uneven plate. Such plates allow for easily defining the operation space in between themselves which can efficiently be evaluated since the well defined and eventually simple geometry. In a specific example, the first conductor element can be provided in form of a roll of a roller compaction for pressing a power mixture of the target pharmaceutical sample into a ribbon, while the second conductor element can be a curved segment, which limits the operation space between both conductor elements.

The controller of the solid fraction sensor can be adapted in any suitable manner for determining the capacitance. For example, it can involve a time based determination in which, typically, an unknown capacitance is used to modify an oscillator circuit frequency. Or, it can involve a bridge determination in which two voltage dividers are compared wherein one path is known and the other one comprises the unknown capacitance.

In a preferred embodiment of the solid fraction sensor, the controller of the solid fraction sensor is adapted to determine the capacitance by a capacitance-to-digital conversion (CDC) and, more specifically, it can be adapted to apply sigma-delta modulation to determine the capacitance.

In another embodiment of the solid fraction sensor, is adapted to measure a discharge time and to determine the capacitance by using the measured discharge time. For example, the solid fraction sensor can be implemented as or comprise a PICO-CAP converter.

In still another preferred embodiment of the solid fraction sensor, the controller is adapted to determine the capacitance by using a charge-balancing circuit or method.

For adjusting the gap between the first and the second conductor element or for adjusting the size of the operation space, respectively, the solid fraction sensor preferably comprises a displacement structure, wherein the at least one of the first conductor element of the solid fraction sensor and the second conductor element of the solid fraction sensor is mounted to the displacement structure such that the first conductor element of the solid fraction sensor and the second conductor element of the solid fraction sensor are movable relative to each other. The displacement structure allows the adjustment of an air-gap in the operation space to a minimum such that the accuracy of the solid fraction determination can be increased or optimized.

The operation space of the solid fraction sensor can be a space in which the first and second conductor elements may generate an electric field. For example, the first and second conductor elements may be positioned aside each other such that the operation space is located above or below the two conductor elements where the electric field can be generated. However, preferably, the solid fraction sensor is embodied such that the operation space of the solid fraction sensor is a gap separating the first conductor element and the second conductor element. Such a gap allows for well defining the operation space which makes the determination of the capacitance comparable simple and efficient.

When determining the solid fraction of the pharmaceutical substance, in general, any geometrical difference, composition difference and moisture content difference between the reference pharmaceutical substance and the pharmaceutical substance should be accounted for in order to achieve a high accuracy. For example, surface pattern, e.g.

caused by ribbons produced with patterned rolls while the reference pharmaceutical substance may be produced without, may occur which can influence the accuracy of the solid fraction determination. Non-variable differences can be accounted for by correction of the measured signal prior comparison with the calibration curve or calibration data. One option is to include as many such dependencies in the multi-variate calibration curve or calibration data as feasible. However, this could be comparably cumbersome as it might cause and extensive calibration requirement.

For those properties that are more or less constant such as, e.g., moisture content, some of the dimensions and the like, the accuracy lowering effects may be reduced by choosing a suitable reference pharmaceutical substance that matches the properties of the pharmaceutical sample and measured at operating conditions. When this is impractical, it might be tried to account for them in the evaluation of the reference pharmaceutical substance such as, e.g., surface pattern can be accounted for instead of forcing the reference pharmaceutical substance having the same surface pattern. When the sample properties varies it might be beneficial to provide active correction by having independent measure of the variable properties. Once such properties are measured, they can be accounted for numerically instead of having multi-variate calibration.

In this context, the pharmaceutical manufacturing installation preferably comprises a thickness measuring unit adapted to measure a thickness of the pharmaceutical substance in its solid state, preferably, when positioned in the operation space of the solid fraction sensor. The thickness measuring unit can be any suitable measurement arrangement such as an electrical, mechanical, optical, acoustic or combined sensor. However, preferably, the thickness measuring sensor comprises a distance capacitance sensor. Such arrangement allows for determining the thickness of the target pharmaceutical sample by the same or similar means of principles applied for determining the capacitance.

Preferably, at least one of the first conductor element of the solid fraction sensor and the second conductor element of the solid fraction sensor is equipped with an insulating layer towards the operation space. Such insulating layer may allow for minimizing effects of parasitic resistivity of the target pharmaceutical sample on the measurement. It may further help to increase the lifetime of the respective conductor element. Also, it may help to prevent contamination of the target pharmaceutical sample. Still further, it may prevent or reduce dust build up on the sensor. Finally, it may also allow for easier cleaning of the sensor and particularly its conductor elements.

Preferably, the solid fraction sensor comprises a reference third conductor element and a reference fourth conductor element together establishing a reference capacitor, wherein the controller of the solid fraction sensor is adapted to being responsive to a difference between an output of a measuring capacitor established by the first conductor element and the second conductor element and an output of the reference capacitor. By providing such reference capacitor the influence of the environmental and operating conditions, such as temperature, humidity or the like, on the sample measurement can be reduced or minimized. In particular, it may allow compensation in situation where the calibration curve does not correspond to the operating conditions and, thus, the sensing may be inaccurate.

Preferably, the first conductor element and/or the second conductor element of the solid fraction sensor has a surface area adjacent to the operation space in a range of between 1 mm$^2$ and 10'000 mm$^2$ or preferably between 10 mm$^2$ and 1'000 mm$^2$.

Preferably, the solid fraction sensor establishes a sensor circuit which operates with a dynamic range of 0 Picofarad (pF) to 1'000 pF, preferably of 0 pF to 100 pF and particularly of 0 pF to 10 pF. The sensor circuit preferably operates with a sensitivity of less than 1'000 Femtofarad (fF), preferrably less than 100 fF and particularly less than 10 fF.

Another aspect of the invention relates to a method of manufacturing a pharmaceutical product comprising the steps of: providing a pharmaceutical substance in a solid state; positioning the pharmaceutical substance in its solid state in an operation space in which an electric field is generated by means of a first conductor element and a second conductor element; determining a capacitance of the pharmaceutical substance in its solid state located in the operation space; and converting the determined capacitance together with information about a composition of a reference pharmaceutical substance having the essentially same dielectric properties as the pharmaceutical substance in its solid state and about a thickness of the reference pharmaceutical substance into a solid fraction of the pharmaceutical substance in its solid state.

The reference pharmaceutical substance can particularly be similar to the pharmaceutical substance wherein, besides the essentially same dielectric properties, the substances can have the same API and/or the essentially same chemistry and/or the essentially same composition.

The method according to the invention and its preferred embodiments described below allow for efficiently achieving the effects and benefits described above in connection with the manufacturing installation according to the invention and the embodiments thereof.

In one preferred embodiment, the pharmaceutical substance in its solid state is bounded. Such a bounded pharmaceutical substance can be a compressed substance such as a tablet, or a ribbon which is further processed to granules or the like, or otherwise bounded such as by lyophilisation. In another preferred embodiment, the pharmaceutical substance in its solid state is unbounded. Such a pharmaceutical substance can, e.g., be a lyophilized powder, any loose powdered material or the like.

Advantageously, the method comprises adjusting a strength of the electric field in the operation space.

Preferably, the information about the composition of the reference pharmaceutical substance comprises permittivity of the reference pharmaceutical substance and a solid fraction ratio of the reference pharmaceutical substance. Such information allow an efficient and accurate evaluation of the solid fraction of the pharmaceutical substance.

Thereby, the information about the composition of the reference pharmaceutical substance and about the thickness of the reference pharmaceutical substance preferably comprises pairs of permittivity and corresponding solid fraction ratio. With such pairs, the permittivity and solid fraction ratio can efficiently be interrelated. In particular, the information about the composition of the reference pharmaceutical substance and about the thickness of the reference pharmaceutical substance preferably is a calibration curve. Such a calibration curve allows for an efficient and reproducible evaluation.

In some applications of the method according to the invention, it can be advantageous to determine the capacitance by a capacitance-to-digital conversion, particularly, by applying a sigma-delta modulation. Like this, a Capacitance to Digital Converter (CDC) or sigma-delta CDC can be involved. With a sigma-delta CDC the method can be realised comparably inexpensive and can have a strong potential as a process analytical technology (PAT) in the pharmaceutical industry.

In other applications, it can be advantageous to measure a discharge time and to determine the capacitance by using the measured discharge time. For example, the measurement of the discharge time can be provided by a PICO CAP converter. Such technique can particularly provide for a suitable accuracy of the capacitance determination.

In still other applications, it can be beneficial to use a charge-balancing circuit to measure the capacitance. Such capacitance measurement can be suitable accurate and fast to be implemented online in a pharmaceutical manufacturing process.

Preferably, the at least one of the first conductor element and the second conductor element is displaced to adjust the operation space. Like this, for example, it can be achieved that the conductor elements preferably slightly contact an object arranged in the operation space. Particularly, the method can comprise a step of adjusting a distance between the first conductor element and the second conductor element such that the first conductor element and the second conductor element contact the pharmaceutical substance in its solid state when positioned in the operation space. Thereby, the occurrence of free space between the conductor elements and the object can be reduced or minimized such that the accuracy of the solid fraction determination can be increased or optimized, since best results of the capacitance measurement may be achieved, when an air gap between the target pharmaceutical sample and one of the first and/or second conductor element is as small as possible.

For an accurate evaluation of the solid fraction, it can be beneficial to further measure a thickness of the target pharmaceutical sample positioned in the operation space.

In a preferred embodiment, the method according to the invention further comprises: positioning the pharmaceutical substance in its solid state in a further operation space of the same or a further solid fraction sensor having a further first conductor element, a further second conductor element, the further operation space and a further energy source arranged to generate an electric field in the further operation space by means of the further first conductor element and the further second conductor element; determining a further capacitance of the pharmaceutical substance in its solid state located in the further operation space; converting the determined further capacitance together with the information about the composition of the reference pharmaceutical substance and about the thickness of the reference pharmaceutical substance into a further solid fraction of the pharmaceutical substance in its solid state, and determining a solid fraction distribution of the solid fraction of the pharmaceutical substance in its solid state and the further solid fraction of the pharmaceutical substance in its solid state. Particularly, when comparably large and/or comparably inhomogeneous pharmaceutical substances are involved, such determination of the solid fraction distribution can be beneficial for achieving a complete or sufficient evaluation of the pharmaceutical substance.

Thereby, the operation space and the further operation space preferably are positioned neighbouring each other. Like this, the solid fraction distribution can be determined by two adjacent capacitors established by the neighbouring operation spaces such that different parts of the pharmaceutical substance can be involved. Also, the solid fraction distribution can be determined by a multi operation space array employing the principles of electrical capacitance tomography.

In a further preferred embodiment, the method according to the invention comprises: positioning the pharmaceutical substance in its solid state in a reference operation space of a reference solid fraction sensor having a reference first conductor element, a reference second conductor element, the reference operation space and a reference energy source arranged to generate an electric field in the reference operation space by means of the reference first conductor element and the reference second conductor element; determining a reference capacitance of the pharmaceutical substance in its solid state located in the reference operation space; converting the determined reference capacitance together with the information about the composition of the reference pharmaceutical substance and about the thickness of the reference pharmaceutical substance into a reference solid fraction of the pharmaceutical substance in its solid state; and comparing the solid fraction of the pharmaceutical substance in its solid state to the reference solid fraction of the pharmaceutical substance in its solid state. Like this, the quality and accuracy of the sensing procedure can be increased.

Brief Description of the Drawings

The pharmaceutical manufacturing installation and the method of manufacturing a pharmaceutical product according to the invention are described in more detail herein below by way of exemplary embodiments and with reference to the attached drawings, in which:

Fig. 1a,b,c    shows an arrangement of a capacitor completely filled with a dielectric, a capacitor partially filled with a dielectric and an equivalent circuit diagram for a theoretical evaluation;

Fig. 2         shows an embodiment of the pharmaceutical manufacturing installation according to the invention for the use with a pharmaceutical substance in form of a tablet;

Fig. 3a,b,c    shows three further embodiments of the pharmaceutical manufacturing installation for the use with a pharmaceutical substance in form of a ribbon compressed out of a powder;

Fig. 4         shows an example of a calibration curve.

Description of Embodiments

To avoid repetition in the figures and the descriptions of the various aspects and illustrative embodiments, it should be understood that many features are common to many aspects and embodiments. Omission of an aspect from a description or figure does not imply that the aspect is missing from embodiments that incorporate that aspect. Instead, the aspect may have been omitted for clarity and to avoid prolix description. In this context, the following applies to the rest of this description: If, in order to clarify the drawings, a figure contains reference signs which are not explained in the directly associated part of the description, then it is referred to previous or following description sections. Further, for reason of lucidity, if in a drawing not all features of a part are provided with reference signs it is referred to other drawings showing the same part. Like numbers in two or more figures represent the same or similar elements.

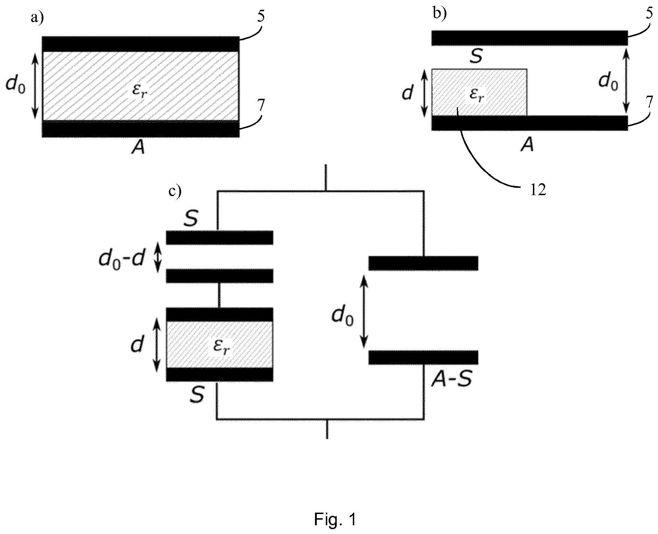

By reference to Fig. 1a,b,c a theoretical evaluation of capacitive sensing is illustrated. Capacitive sensing is a non-contact sensing widely used in many industries, including automotive, oil and gas, medical diagnostics or consumer electronics, and pharmaceutical manufacturing. In general, capacitive sensing is applicable to both conductors and non-conductors. It finds typical use as proximity and displacement sensors. Capacitive sensors are rather inexpensive, especially in comparison to spectroscopy systems, and their simple electronic nature makes them adept for online /inline implementation in manufacturing processes.

Capacitive sensing is also suitable to characterize non-conductive material properties, i.e. dielectrics. Material passing through the gap of the capacitive sensor changes the capacitance of the sensor. When the gap in the capacitor is kept constant, the sensor output will be linked to the change in the thickness, density or composition of the material. If two of these properties are kept constant, the third can be deducted from the measurement. Thus, having a material of homogeneous composition and thickness, its density can be deducted from the sensor output. With a simple calibration, this can be converted into the porosity of the material.

Fig. 1a shows an arrangement of a capacitor completely filled with a dielectric 12 between a first conductor element 5 - in the following also called electrode 5 - and a second conductor element 7 - in the following also called electrode 7. Both electrodes 5, 7 have the same surface size A like the dielectric between them, which dielectric has a thickness of $d_0$ and a permittivity of $\varepsilon_r$. The capacitance $C$ of simple parallel plates is governed by

$$C = \frac{\varepsilon_0 \varepsilon_r A}{d_0} \qquad \text{(Eq. 1)}$$

Here $\varepsilon_0$ is the permittivity of vacuum ($\varepsilon_0$ = 8.85149 pF/m), $\varepsilon_r$ is the relative permittivity of a material between electrodes ($\varepsilon_r$ = 1 for air), A is the surface area of the electrodes and $d_0$ is the distance between the electrodes 5, 7. In order to evaluate the relative permittivity of the material of interest, namely the dielectric, one would normally first obtain the capacitance $C_0$ of empty sensor and capacitance C of sensor fully filled with the material of interest. From the difference between these two, $\Delta C = C - C_0$, one can express the relative permittivity of the material as:

$$\varepsilon_r = 1 + \frac{\Delta C d_0}{\varepsilon_0 A} \qquad \text{(Eq. 2)}$$

If the dielectric sample fills the full thickness of the sensor but does not cover the full area A (not shown), the resulting sensor can be represented by two capacitors in series, one filled with vacuum (air) and another with the sample. The change in capacity is influenced only by the covered surface area S (corresponding to sample surface area), hence one can simply adapt Eq. 2 as

$$\varepsilon_r = 1 + \frac{\Delta C d_0}{\varepsilon_0 S} \qquad \text{(Eq. 3)}$$

It is to note that, within the approximation of homogeneous electric field between the electrodes (i.e. far from the sensor edges), the position of the sample on the electrode does not matter.

A further generalization is necessary in case that the sample does not fill the full thickness of the sensor, as shown in Fig. 1b. The resulting air gap can be represented by two capacitors in series, one filled with air and another with the material of interest, see also Fig.1c with the corresponding equivalent circuit diagram. It is beneficial to define thickness fraction, where $d$ corresponds to the sample thickness. The relative permittivity of the material with thickness $d < d_0$ can be then expressed as

$$\varepsilon_r = 1 + \left( \frac{d}{d_0} \frac{\varepsilon_0 S}{\Delta C d_0} + \frac{d}{d_0} - 1 \right)^{-1} \qquad \text{(Eq. 4)}$$

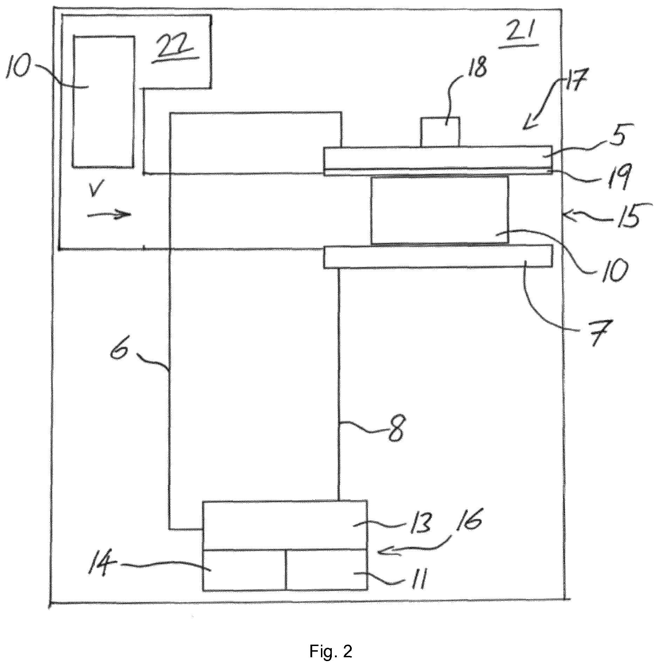

With a view to Fig. 2 the following sensitivity estimation can be determined. A change in sample porosity will result in change in the sample relative permittivity. The non-trivial part is finding a suitable circuitry that allows sensitive enough detection of changes in the relative permittivity. For example, consider a sensor with 3 mm gap between the electrodes 5 and 7 being filled with a 10 mm diameter and 2 mm thick nonporous tablet made of microcrystalline cellulose, which has relative permittivity $\varepsilon_r$ = 5.6 at 58 % relative humidity and 22°C. Using Eq. 1, the increase in the sensor capacitance in the presence of tablet would be approximately 1.6 pF. If a drop in the solid fraction results in a drop of relative permittivity by e.g. 1%, the expected drop in the sensor capacitance would be approx. 20 fF. Hence, the sensing circuit has to be able to detect the capacitance with a few fF accuracy for any practical application as a porosity sensor for solid dosage forms.

Fig. 2 shows as a unit a pharmaceutical installation according to the invention comprising a processing equipment 21 providing a pharmaceutical substance 10 in a solid state and comprising a solid fraction sensor 17. Additionally, a punch arrangement 22 for generating tablets 10 out of the pharmaceutical substance is part of this unit.

In Fig. 2 the pharmaceutical substance is a tablet 10, which is located in the operation space 15 between a first conductor element 5 and a second conductor element 7 of the solid fraction sensor 17. An energy source 13 is connected to the first and second conductor element 5, 7 via a cable 6 respectively via a cable 8. A controller 11 is adapted to adjust a strength of the electric field in the operation space 15 and furthermore, the controller 11 has a data storage 14 in which calibration data is stored. The data storage 14 can be any suitable permanent or volatile data storage such as, e.g., a flash memory, a hard disk, a memory chip, an external storage or cloud storage, or the like.

According to the invention the controller 11 can be adapted to determine the capacitance by a capacitance-to-digital conversion based on the charge-balancing method in combination with the known sigma-delta modulation.

Alternatively, the solid fraction sensor 17 adapted to measure a discharge time and to determine the capacitance by using the measured discharge time, wherein the solid fraction sensor 17 can be implemented as or comprise a PICO-CAP converter.

Furthermore, the solid fraction sensor comprises a displacement structure 18, wherein at least one of the first conductor element 5 and the second conductor element 7 is mounted to the displacement structure 18 such that the first conductor element 5 and the second conductor element 7 are movable relative to each other. By moving the first conductor element 5 and the second conductor 7 relative to each other, the size of the operation space 15 can be adjusted. For example, it can be achieved that the conductor elements preferably slightly contact an object arranged in the operation space 15. Thereby, the occurrence of free space, namely the air gap between the conductor elements 5, 7 and the tablet 10 can be reduced or minimized such that the accuracy of the solid fraction determination can be increased or optimized.

A thickness measuring unit (not shown in the Fig.) is adapted to measure a thickness of the tablet 10 positioned in the operation space 15, wherein the thickness measuring sensor comprises a distance capacitance sensor.

The first conductor element 5 and the second conductor element 7 is equipped with an insulating layer 19 towards the operation space 15 for minimizing effects of parasitic resistivity of the tablet 10 on the measurement. It may further help to increase the lifetime of the respective conductor element 5, 7. Also, it may help to prevent contamination of the tablet 10. Still further, it may prevent or reduce dust build up on the solid fraction sensor 17. Finally, it may also allow for easier cleaning of the solid fraction sensor 17 and particularly its conductor elements 5, 7.

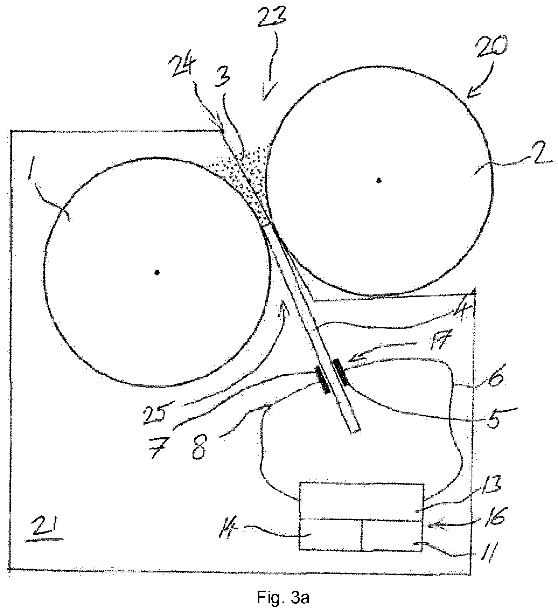

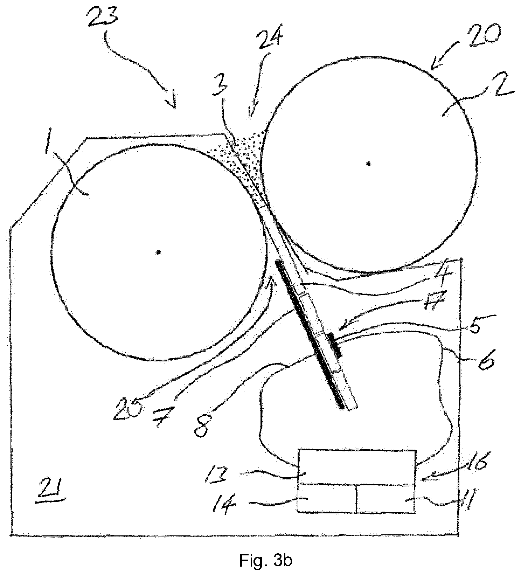

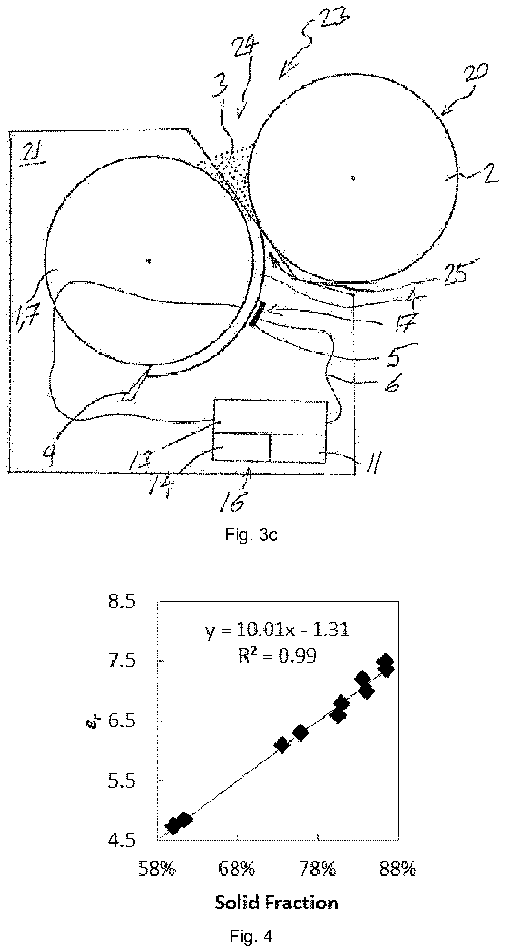

In Fig. 3a,b,c another intended application of the solid fraction sensor 17 in embodiments of pharmaceutical manufacturing installations is shown in the measurement of ribbons 4 prepared by roller compaction before they are milled. A typical roller compaction contains two rolls 1 and 2 which press powder 3 into a ribbon 4. The ribbon 4 is then milled into granules. The solid fraction of the ribbon 4 influences both hardness and size of the granules. It is therefore highly relevant to the bioavailability of the final pharmaceutical products via dissolution and disintegration characteristics. As revealed in all Fig. 3a,b,c, an overall pharmaceutical manufacturing installation 23 comprises a processing equipment 21 arranged to provide a pharmaceutical substance 4 in a solid state and a solid fraction sensor 17. Part of this processing equipment 21 is additionally a first conductor element 5, a second conductor element 7, an operation space 15, an energy source 13 arranged to generate an electric field in the operation space 15 by means of the first conductor element 5 and the second conductor element 7, and a controller 11 adapted to determine a capacitance between the first and second conductor element 5, 7 with the pharmaceutical substance 4 in the solid state located in the operation space 15, wherein the solid fraction sensor 17 is arranged to receive the pharmaceutical substance 4 in its solid state into the operation space 15 by the processing equipment 21. Through a powder inlet zone 24 powder 3 is fed to the processing equipment 21.

In Fig. 3a,b,c a possible implementation of the solid fraction sensor 17 within the roller compactor 20 is outlined. In an ideal case, a representative sample of ribbon 4 is produced without being stuck or keyed to any of the rolls 1, 2. In such circumstances, a similar solid fraction sensor 17 to one shown in Figure 2 can be used and the ribbon 4 can be fed between the electrodes 5, 7 as shown in Fig. 3a.

In practice, the ribbons 4 may not always be strong enough and break. In such cases, the ground electrode 7 can be extended and serve as a support 26, as shown in Fig. 3b. Alternatively, a mechanical support to collect and guide the ribbon 4 can be added to the design, with the electrode 7 implemented within such support. The sensing area will be defined by the solid fraction sensor electrode 5.

When a collar is applied to the fixed roll 1, the ribbons 4 have a strong tendency to remain keyed to the fixed roll 1 and have to be scrapped off by a scraper 9 as shown in Fig. 3c. In such cases, the fixed roll 1 can be used as electrode 7 and the solid fraction sensor requires only one custom-made electrode 5. The sensing area will be again defined by the sensor electrode 5. In this case, the sensor electrode 5 may be curved to limit the inhomogeneity in the generated electric field.

In all cases, the solid fraction sensor 17 can be connected as a floating sensor (with ground electrode floating) or as a grounded sensor (with ground electrode grounded). When the ground electrode is connected as a floating electrode, one of the electrodes can be used for the excitation and another for the read-out. When ground electrode is grounded, the setup requires a switch (not shown) to allow for use of the sensor electrode for both excitation and read-out. The latter is practically useful for the cases described in Fig. 3b and 3c. Here either the support or the roll should be grounded to minimize the parasitic capacitive and resistive signals from the machinery and other external disturbances.

Fig. 4 shows an example of a calibration curve in which pairs of permittivity and corresponding solid fraction ratio of a reference pharmaceutical substance are displayed. In particular, in the example calibration curve, a calibration obtained at uniform operating conditions on tablets with different thickness after thickness correction is shown.

Furthermore, in the claims the word "comprising" does not exclude other elements or steps, and the indefinite article "a" or "an" does not exclude a plurality. A single unit or step may fulfil the functions of several features recited in the claims. The mere fact that certain measures are recited in mutually different dependent claims does not indicate that a combination of these measures cannot be used to advantage. The terms "essentially", "about", "approximately" and the like in connection with an attribute or a value particularly also define exactly the attribute or exactly the value, respectively. The term "about" in the context of a given numerate value or range refers to a value or range that is, e.g., within 20%, within 10%, within 5%, or within 2% of the given value or range. Components described as coupled or connected may be electrically or mechanically directly coupled, or they may be indirectly coupled via one or more intermediate components. Any reference signs in the claims should not be construed as limiting the scope.

## Claims

1. Pharmaceutical manufacturing installation (23), comprising a processing equipment (21) arranged to provide a pharmaceutical substance (3, 4; 10) in a solid state, and a solid fraction sensor (17) having

   a first conductor element (5), a second conductor element (7), an operation space (15), an energy source (13) arranged to generate an electric field in the operation space (15) by means of the first conductor element (6) and the second conductor element (8), and a controller (11) adapted to determine a capacitance between the first and second conductor elements (5, 7) with the pharmaceutical substance (3, 4; 10) in the solid state located in the operation space (15), wherein

the solid fraction sensor (17) is arranged to receive the pharmaceutical substance (3, 4; 10) in its solid state into the operation space (15) by the processing equipment (21).

2. The pharmaceutical manufacturing installation (23) of claim 1, wherein the processing equipment (21) comprises a punch arrangement (22) adapted to generate a tablet (10) of the pharmaceutical substance (3, 4; 10).

3. The pharmaceutical manufacturing installation (23) of claim 1 or 2, wherein one of the first conductor element (6) and the second conductor element (8) is a part of the punch arrangement (22) of the processing equipment (21) or of a tablet chute of the processing equipment (21).

4. The pharmaceutical manufacturing installation (23) of claim 1, wherein

the processing equipment (21) comprises two rotatable rolls (1, 2) and a compacting space in between the rolls (1, 2),
the compacting space has a powder inlet zone (24) and a ribbon outlet zone (25), and
the solid fraction sensor (17) is arranged adjacent to the ribbon outlet zone (25) of the compacting space such that a ribbon (4) generated by the two rolls (1, 2) exiting the compacting space is forwarded into the operation space of the solid fraction sensor, wherein preferably
the second conductor element (7) is formed as a ribbon support (26) adapted to guide a ribbon exiting the compacting space, and/or
one of the two rolls (5, 7) is the second conductor element (7), wherein the first conductor element (5) preferably is bent to correspond to the outer surface of the one of the two rolls (5, 7).

5. The pharmaceutical manufacturing installation (23) of any one of the preceding claims,

wherein the operation space (15) of the solid fraction sensor (17) is a gap separating the first conductor element (5) and the second conductor element (7),
wherein the first conductor element (5) of the solid fraction sensor (17) and the second conductor element (7) of the solid fraction sensor (17) are metallic and plate-like shaped, and/or
wherein a charge-balancing circuit is used to measure the capacitance.

6. The pharmaceutical manufacturing installation (23) of any one of the preceding claims, wherein

the controller (11) of the solid fraction sensor (17) comprises calibration data of a reference pharmaceutical substance having the essentially same dielectric properties as the pharmaceutical substance (3, 4; 10) in its solid state,
the calibration data of the controller (11) of the solid fraction sensor (17) comprises composition data about the composition of the reference pharmaceutical substance and thickness data about the thickness of the reference pharmaceutical substance,
the controller (11) of the solid fraction sensor (17) is adapted to convert the calibration data and the determined capacitance into solid fraction data of the pharmaceutical substance (3, 4; 10) in its solid state, and
the controller (11) is adapted to generate a solid fraction signal representing the solid fraction data.

7. The pharmaceutical manufacturing installation (23) of any one of the preceding claims, wherein

the energy source (13) of the solid fraction sensor (17) is connected to at least one of the first conductor element (5) and the second conductor element (7), and/or
at least one of the first conductor element (5) of the solid fraction sensor (17) and the second conductor element (7) of the solid fraction sensor (17) is equipped with an insulating layer towards the operation space (15).

8. The pharmaceutical manufacturing installation (23) of any one of the preceding claims, wherein the controller (11) of the solid fraction sensor (17)

is adapted to adjust a strength of the electric field in the operation space (15),
is adapted to measure a discharge time and to determine the capacitance by using the measured discharge time,
has a data storage (14) in which the calibration data is stored, and/or

is adapted to determine the capacitance by a capacitance-to-digital conversion, wherein the controller (11) of the solid fraction sensor (17) preferably is adapted to apply sigma-delta modulation to determine the capacitance.

9. The pharmaceutical manufacturing installation (23) of any one of the claims 6 to 8, wherein the calibration data comprises permittivity of the reference pharmaceutical substance and a solid fraction ratio of the reference pharmaceutical substance, wherein the calibration data preferably comprises pairs of permittivity and corresponding solid fraction ratio.

10. The pharmaceutical manufacturing installation (23) of any one of the preceding claims, comprising

   a displacement structure (18), wherein at least one of the first conductor element (5) of the solid fraction sensor (17) and the second conductor element (7) of the solid fraction sensor (17) is mounted to the displacement structure (18) such that the first conductor element (5) of the solid fraction sensor (17) and the second conductor element (5) of the solid fraction sensor (17) are movable relative to each other, and/or
   a thickness measuring unit adapted to measure a thickness of the pharmaceutical substance (3, 4; 10) in its solid state, preferably, when positioned in the operation space of the solid fraction sensor, wherein the thickness measuring sensor preferably comprises a distance capacitance sensor.

11. Method of manufacturing a pharmaceutical product (4, 10) using the pharmaceutical manufacturing installation (23) of any one of claims 1 to 10, comprising the steps of

   providing a pharmaceutical substance (3, 4; 10) in a solid state,
   positioning the pharmaceutical substance (3, 4; 10) in its solid state in an operation space (15) in which an electric field is generated by means of a first conductor element (5) and a second conductor element (7),
   determining a capacitance of the pharmaceutical substance (3, 4; 10) in its solid state located in the operation space (15), and
   converting the determined capacitance together with information about a composition of a reference pharmaceutical substance having the essentially same dielectric properties as the pharmaceutical substance (3, 4; 10) in its solid state and about a thickness of the reference pharmaceutical substance into a solid fraction of the pharmaceutical substance (3, 4; 10) in its solid state.

12. The method of claim 11,

   wherein the pharmaceutical substance (3, 4; 10) in its solid state is bounded or unbounded,
   wherein the capacitance is determined by a capacitance-to-digital conversion,
   wherein a sigma-delta modulation is applied to determine the capacitance,
   wherein a discharge time is measured and the capacitance is determined by using the measured discharge time,
   wherein a charge-balancing circuit is used to measure the capacitance,
   wherein the at least one of the first conductor element and the second conductor element is displaced to adjust the operation space, and/or
   wherein the information about the composition of the reference pharmaceutical substance comprises permittivity of the reference pharmaceutical substance and a solid fraction ratio of the reference pharmaceutical substance, wherein the information about the composition of the reference pharmaceutical substance and about the thickness of the reference pharmaceutical substance preferably comprises pairs of permittivity and corresponding solid fraction ratio, wherein the information about the composition of the reference pharmaceutical substance and about the thickness of the reference pharmaceutical substance preferably is a calibration curve.

13. The method of claim 11 or 12, comprising

   adjusting a strength of the electric field in the operation space (15),
   measuring a thickness of the target pharmaceutical sample positioned in the operation space, and/or
   a step of adjusting a distance between the first conductor element (5) and the second conductor element (7) such that the first conductor element (5) and the second conductor element (7) contact the pharmaceutical substance (3, 4; 10) in its solid state when positioned in the operation space (15).

14. The method of any one of claims 11 to 13, comprising

   positioning the pharmaceutical substance (3, 4; 10) in its solid state in a further operation space of the solid fraction sensor or a further solid fraction sensor, having a further first conductor element (5), a further second conductor element (7), the further operation space (15) and a further energy source (13) arranged to generate an electric field in the further operation space (15) by means of the further first conductor element (5) and the further second conductor element (7),

determining a further capacitance of the pharmaceutical substance (3, 4; 10) in its solid state located in the further operation space (15),

converting the determined further capacitance together with the information about the composition of the reference pharmaceutical substance and about the thickness of the reference pharmaceutical substance into a further solid fraction of the pharmaceutical substance (3, 4; 10) in its solid state, and

determining a solid fraction distribution of the solid fraction of the pharmaceutical substance (3, 4; 10) in its solid state and the further solid fraction of the pharmaceutical substance (3, 4; 10) in its solid state.

15. The method of any one of claims 11 to 14, comprising

positioning the pharmaceutical substance in its solid state in a reference operation space of a reference solid fraction sensor having a reference first conductor element, a reference second conductor element, the reference operation space and a reference energy source arranged to generate an electric field in the reference operation space by means of the reference first conductor element and the reference second conductor element,

determining a reference capacitance of the pharmaceutical substance in its solid state located in the reference operation space,

converting the determined reference capacitance together with the information about the composition of the reference pharmaceutical substance and about the thickness of the reference pharmaceutical substance into a reference solid fraction of the pharmaceutical substance in its solid state, and

comparing the solid fraction of the pharmaceutical substance in its solid state to the reference solid fraction of the pharmaceutical substance in its solid state.

**Patentansprüche**

1. Pharmazeutische Herstellungsanlage (23), die eine Verarbeitungsausrüstung (21), die zum Bereitstellen einer pharmazeutischen Substanz (3, 4; 10) in einem festen Zustand angeordnet ist, und einen Feststoffgehaltssensor (17) umfasst, der

ein erstes Leiterelement (5), ein zweites Leiterelement (7), einen Ablaufraum (15), eine Energiequelle (13), die zum Erzeugen eines elektrischen Felds im Ablaufraum (15) mittels des ersten Leiterelements (6) und des zweiten Leiterelements (8) angeordnet ist, und eine Steuerung (11) aufweist, die zum Bestimmen einer Kapazität zwischen dem ersten und dem zweiten Leiterelement (5, 7) mit der sich im Ablaufraum (15) befindenden pharmazeutischen Substanz (3, 4; 10) im festen Zustand angepasst ist, wobei

der Feststoffgehaltssensor (17) angeordnet ist, um die pharmazeutische Substanz (3, 4; 10) in ihrem festen Zustand in den Ablaufraum (15) durch die Verarbeitungsausrüstung (21) aufzunehmen.

2. Pharmazeutische Herstellungsanlage (23) nach Anspruch 1, wobei die Verarbeitungsausrüstung (21) eine Stanzanordnung (22) umfasst, die angepasst ist, um eine Tablette (10) der pharmazeutischen Substanz (3, 4; 10) zu erzeugen.

3. Pharmazeutische Herstellungsanlage (23) nach Anspruch 1 oder 2, wobei das erste Leiterelement (6) oder das zweite Leiterelement (8) ein Teil der Stanzanordnung (22) der Verarbeitungsausrüstung (21) oder einer Tablettenrutsche der Verarbeitungsausrüstung (21) ist.

4. Pharmazeutische Herstellungsanlage (23) nach Anspruch 1, wobei

die Verarbeitungsausrüstung (21) zwei drehbare Walzen (1, 2) und einen Verdichtungsraum zwischen den Walzen (1, 2) umfasst,

der Verdichtungsraum eine Pulvereinlasszone (24) und eine Bandauslasszone (25) aufweist, und

der Feststoffgehaltssensor (17) derart angrenzend an die Bandauslasszone (25) des Verdichtungsraums angeordnet ist, dass ein Band (4), das durch die zwei Walzen (1, 2) erzeugt wird, die aus dem Verdichtungsraum austreten, in den Ablaufraum des Feststoffgehaltssensors geleitet wird, wobei vorzugsweise

das zweite Leiterelement (7) als ein Bandträger (26) ausgebildet ist, der angepasst ist, um ein Band, das aus dem Verdichtungsraum austritt, zu führen, und/oder
eine der zwei Walzen (5, 7) das zweite Leiterelement (7) ist, wobei das erste Leiterelement (5) vorzugsweise gebogen ist, um der äußeren Oberfläche der einen der zwei Walzen (5, 7) zu entsprechen.

**5.** Pharmazeutische Herstellungsanlage (23) nach einem der vorhergehenden Ansprüche,

wobei der Ablaufraum (15) des Feststoffgehaltssensors (17) eine Lücke ist, die das erste Leiterelement (5) und das zweite Leiterelement (7) trennt,
wobei das erste Leiterelement (5) des Feststoffgehaltssensors (17) und das zweite Leiterelement (7) des Feststoffgehaltssensors (17) metallisch und plattenförmig geformt sind, und/oder
wobei eine Ladungsausgleichsschaltung verwendet wird, um die Kapazität zu messen.

**6.** Pharmazeutische Herstellungsanlage (23) nach einem der vorhergehenden Ansprüche, wobei

die Steuerung (11) des Feststoffgehaltssensors (17) Kalibrierdaten einer pharmazeutischen Referenzsubstanz umfasst, die im Wesentlichen die gleichen dielektrischen Eigenschaften wie die pharmazeutische Substanz (3, 4; 10) in ihrem festen Zustand aufweist,
die Kalibrierdaten der Steuerung (11) des Feststoffgehaltssensors (17) Zusammensetzungsdaten über die Zusammensetzung der pharmazeutischen Referenzsubstanz und Dickendaten über die Dicke der pharmazeutischen Referenzsubstanz umfassen,
die Steuerung (11) des Feststoffgehaltssensors (17) angepasst ist, um die Kalibrierdaten und die bestimmte Kapazität in Feststoffgehaltsdaten der pharmazeutischen Substanz (3, 4; 10) in ihrem festen Zustand umzuwandeln, und
die Steuerung (11) angepasst ist, um ein Feststoffgehaltssignal zu erzeugen, das die Feststoffgehaltsdaten repräsentiert.

**7.** Pharmazeutische Herstellungsanlage (23) nach einem der vorhergehenden Ansprüche, wobei

die Energiequelle (13) des Feststoffgehaltssensors (17) mit dem ersten Leiterelement (5) und/oder dem zweiten Leiterelement (7) verbunden ist, und/oder
wenigstens das erste Leiterelement (5) des Feststoffgehaltssensors (17) und/oder das zweite Leiterelement (7) des Feststoffgehaltssensors (17) mit einer isolierenden Schicht zu dem Ablaufraum (15) hin ausgestattet ist.

**8.** Pharmazeutische Herstellungsanlage (23) nach einem der vorhergehenden Ansprüche, wobei die Steuerung (11) des Feststoffgehaltssensors (17)

angepasst ist, um eine Stärke des elektrischen Feldes im Ablaufraum (15) einzustellen,
angepasst ist, um eine Entladungszeit zu messen und die Kapazität durch Verwenden der gemessenen Entladungszeit zu bestimmen,
einen Datenspeicher (14) aufweist, in dem die Kalibrierdaten gespeichert sind, und/oder
angepasst ist, um die Kapazität durch eine Kapazität-zu-Digital-Wandlung zu bestimmen, wobei die Steuerung (11) des Feststoffgehaltssensors (17) vorzugsweise angepasst ist, um eine Sigma-Delta-Modulation anzuwenden, um die Kapazität zu bestimmen.

**9.** Pharmazeutische Herstellungsanlage (23) nach einem der Ansprüche 6 bis 8, wobei die Kalibrierdaten eine Permittivität der pharmazeutischen Referenzsubstanz und ein Feststoffgehaltsverhältnis der pharmazeutischen Referenzsubstanz umfassen, wobei die Kalibrierdaten vorzugsweise Paare von Permittivität und entsprechendem Feststoffgehaltsverhältnis umfassen.

**10.** Pharmazeutische Herstellungsanlage (23) nach einem der vorhergehenden Ansprüche, die Folgendes umfasst:

eine Verschiebungsstruktur (18), wobei zumindest eines des ersten Leiterelements (5) des Feststoffgehaltssensors (17) oder des zweiten Leiterelements (7) des Feststoffgehaltssensors (17) an der Verschiebungsstruktur (18) derart montiert ist, dass das erste Leiterelement (5) des Feststoffgehaltssensors (17) und das zweite Leiterelement (5) des Feststoffgehaltssensors (17) relativ zueinander bewegbar sind, und/oder
eine Dickenmesseinheit, die angepasst ist, um eine Dicke der pharmazeutischen Substanz (3, 4; 10) in ihrem festen Zustand zu messen, vorzugsweise, wenn sie im Ablaufraum des Feststoffgehaltssensors positioniert ist, wobei der Dickenmesssensor vorzugsweise einen Abstandskapazitätssensor umfasst.

**11.** Verfahren zum Herstellen eines pharmazeutischen Produkts (4, 10) unter Verwendung der pharmazeutischen Herstellungsanlage (23) nach einem der Ansprüche 1 bis 10, das die folgenden Schritte umfasst:

Bereitstellen einer pharmazeutischen Substanz (3, 4; 10) in einem festen Zustand,
Positionieren der pharmazeutischen Substanz (3, 4; 10) in ihrem festen Zustand in einem Ablaufraum (15), in dem ein elektrisches Feld mittels eines ersten Leiterelements (5) und eines zweiten Leiterelements (7) erzeugt wird,
Bestimmen einer Kapazität der pharmazeutischen Substanz (3, 4; 10) in ihrem festen Zustand, die sich in dem Ablaufraum (15) befindet, und
Umwandeln der bestimmten Kapazität zusammen mit Informationen über eine Zusammensetzung einer pharmazeutischen Referenzsubstanz, die im Wesentlichen die gleichen dielektrischen Eigenschaften wie die pharmazeutische Substanz (3, 4; 10) in ihrem festen Zustand aufweist, und über eine Dicke der pharmazeutischen Referenzsubstanz in einen Feststoffgehalt der pharmazeutischen Substanz (3, 4; 10) in ihrem festen Zustand.

**12.** Verfahren nach Anspruch 11,

wobei die pharmazeutische Substanz (3, 4; 10) in ihrem festen Zustand gebunden oder ungebunden ist,
wobei die Kapazität durch eine Kapazität-zu-Digital-Wandlung bestimmt wird,
wobei eine Sigma-Delta-Modulation angewendet wird, um die Kapazität zu bestimmen,
wobei eine Entladungszeit gemessen wird und die Kapazität durch Verwenden der gemessenen Entladungszeit bestimmt wird,
wobei eine Ladungsausgleichsschaltung verwendet wird, um die Kapazität zu messen,
wobei das erste Leiterelement und/oder das zweite Leiterelement verschoben wird, um den Ablaufraum einzustellen, und/oder
wobei die Informationen über die Zusammensetzung der pharmazeutischen Referenzsubstanz die Permittivität der pharmazeutischen Referenzsubstanz und ein Feststoffgehaltsverhältnis der pharmazeutischen Referenzsubstanz umfassen, wobei die Informationen über die Zusammensetzung der pharmazeutischen Referenzsubstanz und über die Dicke der pharmazeutischen Referenzsubstanz vorzugsweise Paare von Permittivität und entsprechendem Feststoffgehaltsverhältnis umfassen, wobei die Informationen über die Zusammensetzung der pharmazeutischen Referenzsubstanz und über die Dicke der pharmazeutischen Referenzsubstanz vorzugsweise eine Kalibrierkurve ist.

**13.** Verfahren nach Anspruch 11 oder 12, das Folgendes umfasst:

Einstellen einer Stärke des elektrischen Feldes in dem Ablaufraum (15),
Messen einer Dicke der pharmazeutischen Zielprobe, die in dem Ablaufraum positioniert ist, und/oder
einen Schritt zum Einstellen eines Abstands zwischen dem ersten Leiterelement (5) und dem zweiten Leiterelement (7), so dass das erste Leiterelement (5) und das zweite Leiterelement (7) die pharmazeutische Substanz (3, 4; 10) in ihrem festen Zustand berühren, wenn sie in dem Ablaufraum (15) positioniert ist.

**14.** Verfahren nach einem der Ansprüche 11 bis 13, das Folgendes umfasst:

Positionieren der pharmazeutischen Substanz (3, 4; 10) in ihrem festen Zustand in einem weiteren Ablaufraum des Feststoffgehaltssensors oder eines weiteren Feststoffgehaltssensors, der ein weiteres erstes Leiterelement (5), ein weiteres zweites Leiterelement (7), den weiteren Ablaufraum (15) und eine weitere Energiequelle (13) aufweist, die angeordnet ist, um ein elektrisches Feld in dem weiteren Ablaufraum (15) mittels des weiteren ersten Leiterelements (5) und des weiteren zweiten Leiterelements (7) zu erzeugen,
Bestimmen einer weiteren Kapazität der pharmazeutischen Substanz (3, 4; 10) in ihrem festen Zustand, die sich in dem weiteren Ablaufraum (15) befindet,
Umwandeln der bestimmten weiteren Kapazität zusammen mit den Informationen über die Zusammensetzung der pharmazeutischen Referenzsubstanz und über die Dicke der pharmazeutischen Referenzsubstanz in einen weiteren Feststoffgehalt der pharmazeutischen Substanz (3, 4; 10) in ihrem festen Zustand, und
Bestimmen einer Feststoffgehaltsverteilung des Feststoffgehalts der pharmazeutischen Substanz (3, 4; 10) in ihrem festen Zustand und des weiteren Feststoffgehalts der pharmazeutischen Substanz (3, 4; 10) in ihrem festen Zustand.

**15.** Verfahren nach einem der Ansprüche 11 bis 14, das Folgendes umfasst:

Positionieren der pharmazeutischen Substanz in ihrem festen Zustand in einem Referenzablaufraum eines Referenzfeststoffgehaltssensors, der ein erstes Referenzleiterelement, ein zweites Referenzleiterelement, den Referenzablaufraum und eine Referenzenergiequelle aufweist, die angeordnet ist, um ein elektrisches Feld in dem Referenzablaufraum mittels des ersten Referenzleiterelements und des zweiten Referenzleiterelements zu erzeugen,

Bestimmen einer Referenzkapazität der pharmazeutischen Substanz in ihrem festen Zustand, die sich in dem Referenzablaufraum befindet,

Umwandeln der bestimmten Referenzkapazität zusammen mit den Informationen über die Zusammensetzung der pharmazeutischen Referenzsubstanz und über die Dicke der pharmazeutischen Referenzsubstanz in einen Referenzfeststoffgehalt der pharmazeutischen Substanz in ihrem festen Zustand, und

Vergleichen des Feststoffgehalts der pharmazeutischen Substanz in ihrem festen Zustand mit dem Referenzfeststoffgehalt der pharmazeutischen Substanz in ihrem festen Zustand.

**Revendications**

1. Installation de fabrication pharmaceutique (23), comprenant un équipement de traitement (21) disposé pour fournir une substance pharmaceutique (3, 4 ; 10) dans un état solide, et un capteur de fraction solide (17) ayant

un premier élément conducteur (5), un second élément conducteur (7), un espace de fonctionnement (15), une source d'énergie (13) disposée pour générer un champ électrique dans l'espace de fonctionnement (15) au moyen du premier élément conducteur (6) et du second élément conducteur (8), et un dispositif de commande (11) adapté pour déterminer une capacité entre les premier et second éléments conducteurs (5, 7) avec la substance pharmaceutique (3, 4 ; 10) dans l'état solide située dans l'espace de fonctionnement (15), le capteur de fraction solide (17) étant disposé pour recevoir la substance pharmaceutique (3, 4 ; 10) dans son état solide dans l'espace de fonctionnement (15) par l'équipement de traitement (21).

2. Installation de fabrication pharmaceutique (23) selon la revendication 1, l'équipement de traitement (21) comprenant une disposition de poinçon (22) adaptée pour générer un comprimé (10) de la substance pharmaceutique (3, 4 ; 10).

3. Installation de fabrication pharmaceutique (23) selon la revendication 1 ou 2, soit le premier élément conducteur (6), soit le second élément conducteur (8) faisant partie de la disposition de poinçon (22) de l'équipement de traitement (21) ou d'une goulotte de l'équipement de traitement (21).

4. Installation de fabrication pharmaceutique (23) selon la revendication 1,

l'équipement de traitement (21) comprenant deux rouleaux rotatifs (1, 2) et un espace de compactage entre les rouleaux (1, 2),
l'espace de compactage ayant une zone d'entrée de poudre (24) et une zone de sortie de ruban (25), et
le capteur de fraction solide (17) étant disposé à proximité de la zone de sortie de ruban (25) de l'espace de compactage de telle sorte qu'un ruban (4) généré par les deux rouleaux (1, 2) sortant de l'espace de compactage est acheminé dans l'espace de fonctionnement du capteur de fraction solide, de préférence
le second élément conducteur (7) étant formé comme un support de ruban (26) adapté pour guider un ruban sortant de l'espace de compactage et/ou
l'un des deux rouleaux (5, 7) étant le second élément conducteur (7), le premier élément conducteur (5) étant de préférence plié pour correspondre à la surface externe de l'un des deux rouleaux (5, 7).

5. Installation de fabrication pharmaceutique (23) selon l'une quelconque des revendications précédentes,

l'espace de fonctionnement (15) du capteur de fraction solide (17) étant un espace séparant le premier élément conducteur (5) et le second élément conducteur (7),
le premier élément conducteur (5) du capteur de fraction solide (17) et le second élément conducteur (7) du capteur de fraction solide (17) étant métalliques et en forme de plaque et/ou
un circuit d'équilibrage de charge étant utilisé pour mesurer la capacité.

6. Installation de fabrication pharmaceutique (23) selon l'une quelconque des revendications précédentes,

le dispositif de commande (11) du capteur de fraction solide (17) comprenant des données d'étalonnage d'une substance pharmaceutique de référence ayant sensiblement les mêmes propriétés diélectriques que la substance pharmaceutique (3, 4 ; 10) dans son état solide,

les données d'étalonnage du dispositif de commande (11) du capteur de fraction solide (17) comprenant des données de composition concernant la composition de la substance pharmaceutique de référence et des données d'épaisseur concernant l'épaisseur de la substance pharmaceutique de référence,

le dispositif de commande (11) du capteur de fraction solide (17) étant adapté pour convertir les données d'étalonnage et la capacité déterminée en données de fraction solide de la substance pharmaceutique (3, 4 ; 10) dans son état solide, et

le dispositif de commande (11) étant adapté pour générer un signal de fraction solide représentant les données de fraction solide.

7.  Installation de fabrication pharmaceutique (23) selon l'une quelconque des revendications précédentes,

la source d'énergie (13) du capteur de fraction solide (17) étant reliée au premier élément conducteur (5) et/ou au second élément conducteur (7), et/ou

le premier élément conducteur (5) du capteur de fraction solide (17) et/ou le second élément conducteur (7) du capteur de fraction solide (17) étant équipés d'une couche isolante vers l'espace de fonctionnement (15).

8.  Installation de fabrication pharmaceutique (23) selon l'une quelconque des revendications précédentes, le dispositif de commande (11) du capteur de fraction solide (17)

étant adapté pour régler une intensité du champ électrique dans l'espace de fonctionnement (15),

étant adapté pour mesurer un temps de décharge et pour déterminer la capacité à l'aide du temps de décharge mesuré,

présentant une mémoire de données (14) dans laquelle les données d'étalonnage sont stockées, et/ou

étant adapté pour déterminer la capacité par une conversion capacité à numérique, le dispositif de commande (11) du capteur de fraction solide (17) étant de préférence adapté pour appliquer une modulation sigma-delta pour déterminer la capacité.

9.  Installation de fabrication pharmaceutique (23) selon l'une quelconque des revendications 6 à 8, les données d'étalonnage comprenant la permittivité de la substance pharmaceutique de référence et un rapport de fraction solide de la substance pharmaceutique de référence, les données d'étalonnage comprenant de préférence des paires de permittivité et un rapport de fraction solide correspondant.

10. Installation de fabrication pharmaceutique (23) selon l'une quelconque des revendications précédentes, comprenant

une structure de déplacement (18), le premier élément conducteur (5) du capteur de fraction solide (17) et/ou le second élément conducteur (7) du capteur de fraction solide (17) étant montés sur la structure de déplacement (18) de telle sorte que le premier élément conducteur (5) du capteur de fraction solide (17) et le second élément conducteur (5) du capteur de fraction solide (17) sont mobiles l'un par rapport à l'autre, et/ou

une unité de mesure d'épaisseur adaptée pour mesurer une épaisseur de la substance pharmaceutique (3, 4 ; 10) dans son état solide, de préférence, lorsqu'elle est positionnée dans l'espace de fonctionnement du capteur de fraction solide, le capteur de mesure d'épaisseur comprenant de préférence un capteur de capacité de distance.

11. Procédé de fabrication d'un produit pharmaceutique (4, 10) à l'aide de l'installation de fabrication pharmaceutique (23) selon l'une quelconque des revendications 1 à 10, comprenant les étapes suivantes

la fourniture d'une substance pharmaceutique (3, 4 ; 10) dans un état solide,

la position de la substance pharmaceutique (3, 4 ; 10) dans son état solide dans un espace de fonctionnement (15) dans lequel un champ électrique est généré au moyen d'un premier élément conducteur (5) et d'un second élément conducteur (7),

la détermination d'une capacité de la substance pharmaceutique (3, 4 ; 10) dans son état solide située dans l'espace de fonctionnement (15), et

la conversion de la capacité déterminée conjointement avec des informations concernant une composition d'une substance pharmaceutique de référence ayant essentiellement les mêmes propriétés diélectriques que la substance pharmaceutique (3, 4 ; 10) dans son état solide et concernant une épaisseur de la substance pharmaceutique de référence en une fraction solide de la substance pharmaceutique (3, 4 ; 10) dans son état solide.

**12.** Procédé selon la revendication 11,

la substance pharmaceutique (3, 4 ; 10) dans son état solide étant limitée ou illimitée,
la capacité étant déterminée par une conversion capacité à numérique,
une modulation sigma-delta étant appliquée pour déterminer la capacité,
un temps de décharge étant mesuré et la capacité étant déterminée à l'aide du temps de décharge mesuré,
un circuit d'équilibrage de charge étant utilisé pour mesurer la capacité,
le premier élément conducteur et/ou le second élément conducteur étant déplacé pour régler l'espace de fonctionnement, et/ou
les informations concernant la composition de la substance pharmaceutique de référence comprenant la permittivité de la substance pharmaceutique de référence et un rapport de fraction solide de la substance pharmaceutique de référence, les informations concernant la composition de la substance pharmaceutique de référence et concernant l'épaisseur de la substance pharmaceutique de référence comprenant de préférence des paires de permittivité et de rapport de fraction solide correspondant, les informations concernant la composition de la substance pharmaceutique de référence et concernant l'épaisseur de la substance pharmaceutique de référence étant de préférence une courbe d'étalonnage.

**13.** Procédé selon la revendication 11 ou 12, comprenant

le réglage d'une intensité du champ électrique dans l'espace de fonctionnement (15),
la mesure d'une épaisseur de l'échantillon pharmaceutique cible positionné dans l'espace de fonctionnement, et/ou
une étape de réglage d'une distance entre le premier élément conducteur (5) et le second élément conducteur (7) de telle sorte que le premier élément conducteur (5) et le second élément conducteur (7) sont en contact avec la substance pharmaceutique (3, 4 ; 10) dans son état solide lorsqu'elle est positionnée dans l'espace de fonctionnement (15).

**14.** Procédé selon l'une quelconque des revendications 11 à 13, comprenant

le positionnement de la substance pharmaceutique (3, 4 ; 10) dans son état solide dans un espace de fonctionnement supplémentaire du capteur de fraction solide ou d'un capteur de fraction solide supplémentaire, ayant un premier élément conducteur supplémentaire (5), un second élément conducteur supplémentaire (7), l'espace de fonctionnement supplémentaire (15) et une source d'énergie supplémentaire (13) disposée pour générer un champ électrique dans l'espace de fonctionnement supplémentaire (15) au moyen du premier élément conducteur supplémentaire (5) et du second élément conducteur supplémentaire (7),
la détermination d'une capacité supplémentaire de la substance pharmaceutique (3, 4 ; 10) dans son état solide située dans l'espace de fonctionnement supplémentaire (15),
la conversion de la capacité supplémentaire déterminée conjointement avec les informations concernant la composition de la substance pharmaceutique de référence et concernant l'épaisseur de la substance pharmaceutique de référence en une fraction solide supplémentaire de la substance pharmaceutique (3, 4 ; 10) dans son état solide, et
la détermination d'une distribution de fraction solide de la fraction solide de la substance pharmaceutique (3, 4 ; 10) dans son état solide et de la fraction solide supplémentaire de la substance pharmaceutique (3, 4 ; 10) dans son état solide.

**15.** Procédé selon l'une quelconque des revendications 11 à 14, comprenant

le positionnement de la substance pharmaceutique dans son état solide dans un espace de fonctionnement de référence d'un capteur de fraction solide de référence ayant un premier élément conducteur de référence, un second élément conducteur de référence, l'espace de fonctionnement de référence et une source d'énergie de référence disposée pour générer un champ électrique dans l'espace de fonctionnement de référence au moyen du premier élément conducteur de référence et du second élément conducteur de référence,
la détermination d'une capacité de référence de la substance pharmaceutique dans son état solide située dans l'espace de fonctionnement de référence,
la conversion de la capacité de référence déterminée conjointement avec les informations concernant la composition de la substance pharmaceutique de référence et concernant l'épaisseur de la substance pharmaceutique de référence en une fraction solide de référence de la substance pharmaceutique dans son état solide, et la comparaison de la fraction solide de la substance pharmaceutique dans son état solide à la fraction solide de référence de la substance pharmaceutique dans son état solide.

Fig. 1

Fig. 2

Fig. 3a

Fig. 3b

Fig. 3c

**Solid Fraction**

Fig. 4

**REFERENCES CITED IN THE DESCRIPTION**

**Patent documents cited in the description**

- DE 2502098